(19) Europäisches Patentamt
European Patent Office
Office européen des brevets

(11) Publication number : 0 430 473 B1

(12) # EUROPEAN PATENT SPECIFICATION

(45) Date of publication of patent specification : 12.01.94 Bulletin 94/02

(51) Int. Cl.$^5$ : A61K 7/42

(21) Application number : 90312248.9

(22) Date of filing : 08.11.90

(54) **Photoprotection compositions having improved efficiency.**

The file contains technical information submitted after the application was filed and not included in this specification

(30) Priority : 29.11.89 US 443595

(43) Date of publication of application : 05.06.91 Bulletin 91/23

(45) Publication of the grant of the patent : 12.01.94 Bulletin 94/02

(84) Designated Contracting States : AT BE CH DE DK ES FR GB GR IT LI LU NL SE

(56) References cited :
EP-A- 0 193 387
EP-A- 0 359 488
GB-A- 2 170 105
US-A- 4 810 489

(56) References cited :
SOAP/COSMETICS/CHEMICAL SPECIALITIES, vol. 63, no. 5, May 1987, pages 28-29,32-33,84-85, New York, US; R.Y. LOCHHEAD et al.: "Hydrophobically modified "carbopol" resins"
Longes handbook of Chemistry p. 2.9 , C.T.F.A International Cosmetic Ingredint Dictionary p 70, 615, 616

(73) Proprietor : Richardson-Vicks, Inc.
Ten Westport Road
Wilton, CT 06897 (US)

(72) Inventor : Thaman, Lauren Ann
3501 Linwood Road
Cincinnati, Ohio 45226 (US)
Inventor : Deckner, George Endel
22 Powder Mill Lane
Trumbull, CT 06611 (US)
Inventor : Sottery, John Phelps
125 Warner Hill Road, Unit 26
Stratford, CT 06497 (US)

(74) Representative : Brooks, Maxim Courtney et al
Procter & Gamble (NTC) Limited Whitley Road
Longbenton
Newcastle-upon-Tyne NE12 9TS (GB)

EP 0 430 473 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to improved topical compositions having improved efficiency such as improved sunscreen effectiveness for protecting the skin from the harmful effects of ultraviolet irradiation, such as sunburn and sun-induced premature aging of the skin.

The damaging effects of sunlight on skin are well documented. Contrary to what most people believe, it is not necessary that one sunbathe to suffer the ill-effects of excessive UV exposure. In fact, a lot of damage can be done just by routine day-to-day activities in the sunlight. Some scientists estimate that over 70 percent of the damage the sun inflicts on the average person's skin over a lifetime is the result of simply being outdoors or even sitting by a window.

The major short term hazard of prolonged exposure to sunlight is erythema (i.e. sunburn). The 290 nm to 320 nm wavelength ultraviolet radiation range, designated as the "UVB" wavelength range, tends to be the primary cause of erythema. The 320 nm to 400 nm wavelength ultraviolet radiation range, designated as the "UVA" wavelength range, also produces erythema.

In addition to the short term hazard or erythema, there are also long term hazards associated with UV radiation exposure. One of these long term hazards is malignant changes in the skin surface. Numerous epidemiologic studies demonstrate a strong relationship between sunlight exposure and human skin cancer.

Another long term hazard of ultraviolet radiation is premature aging of the skin. This condition is characterized by wrinkling and yellowing of the skin, along with other physical changes such as cracking, telangiectasis (spider vessels), solar keratoses (growths), ecchymoses (subcutaneous hemorrhagic lesions), and loss of elasticity (sagging). The adverse effects associated with exposure to UVA and UVB wavelength radiation are more fully discussed in DeSimone, "Sunscreen and Suntan Products", Handbook of Nonprescription Drugs, 7th Ed., Chapter 26, pp. 499-511 (American Pharmaceutical Association, Washington, D.C.; 1982); Grove and forbes, "A Method for Evaluating the Photoprotection Action of Sunscreen Agents Against UV-A Radiation", International Journal of Cosmetic Science, 4, pp. 15-24 (1982); and US-A-4,387,089, DePolo, issued June 7, 1983. Hence, although the immediate effects of ultraviolet radiation may be cosmetically and socially gratifying, the long-term hazards are cumulative and potentially serious. It has been estimated that eighty percent of lifetime sun exposure occurs during multiple brief exposures not intended to produce tanning. Therefore, photoprotection during these exposures to ultraviolet radiation is necessary.

The fact that these effects are taken seriously by the general public is suggested by considering the sun protection products' market. This market has grown considerably in recent years and many new products are introduced each year. What used to be looked upon as a seasonal business is no longer. Sun protection compounds are now included in a diversity of personal care products, particularly cosmetic-type products which are worn on a daily basis.

Sunblock agents are commercially available to protect the skin from UV radiation. These agents scatter or reflect ultraviolet radiation. Examples include titanium dioxide and zinc oxide. However, compositions containing a high level of these agents are opaque, generally unattractive in color, and are viewed as unacceptable for usage on more than just the nose or tops of the ears. Furthermore, these agents are very susceptible to rub-off or wear-off resulting in little or no protection.

The most common agents for sun protection are sunscreens. These agents exert their effects through chemical means, i.e., they absorb ultraviolet radiation so that it cannot penetrate the skin. Sunscreens present the user with several problems. For example, they must be on the surface of the skin at the time of exposure to be effective. Sunscreens are preventative so one must anticipate being in the sun. To be most effective, sunscreens must be on the skin as a continuous uniform film. Delivering such a film to the surface of the skin is very difficult.

Sunscreen-containing oil-in-water emulsions are the most popular form of photoprotection products in the U.S. market. This form is cosmetically pleasing, safe, cost effective and versatile. In general, however, oil-in-water emulsions containing oil-soluble sunscreens do not provide as good sunscreening efficiency when compared to water-in-oil emulsions containing oil soluble sunscreens. Unfortunately, water-in-oil emulsions, while providing good sunscreening efficiency, are at a disadvantage in terms of their cosmetic properties. It is therefore highly desirable to formulate oil-in-water photoprotection emulsions which possess superior cosmetic properties and improved sunscreening efficiency.

Without being limited by theory, it is believed that the specific oil-in-water dispersions of the present invention having a surface tension of greater than 0.04 N/m (40 dynes/cm) and which possess a wax component having a required HLB between 1 and 8 and an oil phase with a required HLB of between 1 and 8, will form continuous oil films when applied to skin and thereby provide increased SPF efficiency. This efficiency can be further enhanced by increasing the viscosity of the residual film and by decreasing its migration propensity on skin.

The compositions of the present invention provide improved film-forming as well as improved skin hydration for improved moisturizing and improved efficiency of the sunscreen active thereby requiring a significantly lower level of sunscreen active than heretofore believed necessary to provide a relatively high level of sunscreen efficacy. The compositions of the present invention further provide reduced sunscreen migration thereby reducing, for example, eye-stinging, by inhibiting the sunscreen active from invading the eye area.

It is therefore an object of the present invention to provide a topical composition in a stable form, the use of which will prevent both acute (erythema) and chronic (photoaging) effects of exposure to the sun.

It is further an object of the present invention to provide specific photoprotection emulsion compositions which provide improved sunscreen efficiency and which have a surface tension of greater than 0.04 N/m (40 dynes/cm) and which possess a wax component having a required HLB between 1 and 8 and an oil phase with a required HLB of between 1 and 8.

It is a still further object of the present invention to provide a photoprotection composition which can be applied to the skin on a daily basis and provide significant photoprotection with relatively lower levels of sunscreen active.

These and other objects will become readily apparent from the detailed description that follows.

According to the present invention there is provided an oil-in-water sunscreen emulsion composition having improved efficiency for topical use comprising:

(a) from 1% to 20% of ethylhexyl-p-methoxycinnamate;

(b) from 0.025% to 2.0% of a carboxylic copolymer comprising polymers of a monomeric mixture monomer selected from acrylic, methacrylic and ethacrylic acids, 1 to 3.5 weight percent of an acrylate ester of the formula:

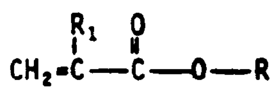

wherein R is hydrogen or an alkyl radical containing 10 to 30 carbon atoms and $R_1$ is hydrogen, methyl or ethyl, and 0.1 to 0.6 weight percent of a polymerizable cross-linking polyalkenyl polyether of a polyhydric alcohol containing more than one alkenyl ether group per molecule wherein the parent polyhydric alcohol contains at least 3 carbon atoms and at least 3 hydroxyl groups;

(c) from 0.1% to 5.0% of a film-forming copolymer of the formula:

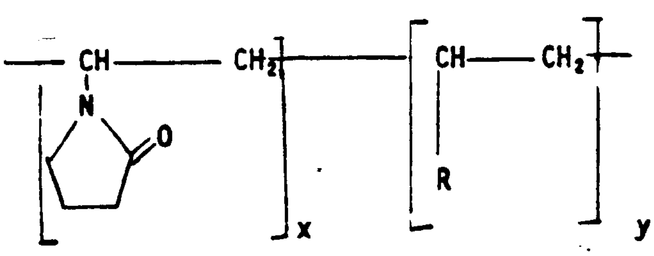

where R is a $C_{12}$-$C_{30}$ straight chain alkyl and wherein the ratio of x to y is from 1:5 to 5:1;

(d) from 0.1% to 10% of a wax component having a required HLB of from 1 to 8 and a melting point greater than 50°C; and

(e) a safe and effective amount of a topical carrier

wherein said sunscreen emulsion has a surface tension greater than 0.04 N/m (40 dynes/cm) preferably greater than 0.042 N/m (42 dynes/cm) and wherein the oil phase has a required HLB between 1 and 8.

All percentages and ratios used herein are by weight of the total composition and all measurements made at 25°C, unless otherwise designated.

In accordance with the present invention, moisture resistant skin treatment compositions, such as sunscreen and sun block formulations, and moisturizer formulations are provided, which compositions have improved efficiency and are in the form of a oil-in-water emulsion which contains water, emollients, emulsifiers, thickeners, preservatives, coloring agents, fragrances, antioxidants and the like and one or more known ultraviolet absorbing compounds (in the case of sunscreen or sun block formulations).

The formulation of the invention is an oil-in-water type emulsion since this type of emulsion affords better cosmetic feel to the product. Depending upon the choice of ingredients, the formulation has a semi-solid cream-like consistency which can be packaged in a plastic squeeze tube or glass jar or it has a lotion type consistency which can be packaged in a plastic squeeze container or bottle. The container can include a flow-type cap or pump-type dispenser.

Sunscreens. The sunscreen useful in the present invention is ethylhexyl-p-methoxycinnamate (available as Parsol (RTM) MCX from Givaudan Corporation).

This sunscreen can be used alone or in combination with a wide variety of conventional sunscreening agents which are suitable for use in the present invention. Segarin, et al., at Chapter VIII, pages 189 et seq., of Cosmetics Science and Technology, disclose numerous suitable agents. Specific suitable sunscreening agents include, for example: p-Aminobenzoic acid, its salts and its derivatives (ethyl, isobutyl, glyceryl esters; p-dimethylaminobenzoic acid); Anthranilates (i.e., o-aminobenzoates; methyl, octyl, amyl, menthyl, phenyl, benzyl, phenylethyl, linalyl, terpinyl, and cyclohexenyl esters); Salicylates (octyl, amyl, phenyl, benzyl, menthyl, glyceryl, and dipropyleneglycol esters); Cinnamic acid derivatives (menthyl and benzyl esters, -phenyl cinnamonitrile; butyl cinnamoyl pyruvate); Dihydroxycinnamic acid derivatives (umbelliferone, methylumbelliferone, methylaceto-umbelliferone); Trihydroxycinnamic acid derivatives (esculetin, methylesculetin, daphnetin, and the glucosides, esculin and daphnin); Hydrocarbons (diphenylbutadiene, stilbene); Dibenzalacetone and benzalacetophenone; Naphtholsulfonates (sodium salts of 2-naphthol 3,6-disulfonic and of 2-naphthol-6,8-disulfonic acids); Dihydroxynaphthoic acid and its salts; o- and p-Hydroxybiphenyldisulfonates; Coumarin derivatives (7-hydroxy, 7-methyl, 3-phenyl); Diazoles (2-acetyl-3-bromoindazole, phenyl benzoxazole, methyl naphthoxazole, various aryl benzothiazoles); Quinine salts (bisulfate, sulfate, chloride, oleate, and tannate); Quinoline derivatives (8-hydroxyquinoline salts, 2-phenylquinoline); Hydroxy- or methoxy-substituted benzophenones; Uric and vilouric acids; Tannic acid and its derivatives (e.g., hexaethylether); (Butyl carbityl) (6-propyl piperonyl) ether; Hydroquinone; Benzophenones (Oxybenzene, Sulisbenzone, Dioxybenzone, Benzoresorcinol, 2,2',4,4'-Tetrahydroxybenzophenone, 2,2'-Dihydroxy-4,4'-dimethoxybenzophenone, Octabenzone; 4-Isopropyldibenzoylmethane; Butylmethoxydibenzoylmethane; Etocrylene; 4-isopropyldi-benzoylmethane; and camphor derivatives such as methyl benzylidene or benzylidene camphor.

A safe and photoprotectively effective amount of sunscreen may be used in the sunscreen compositions of the present invention. By "safe and photoprotectively" is meant an amount sufficient to provide photoprotection when the composition is applied not so much as to cause any side effects or skin reactions. Generally, the additional sunscreen agent may comprise from 1% to 30%, preferably from 2% to 20%, of the composition, Exact amounts will vary depending upon the sunscreen chosen and the desired Sun Protection Factor (SPF).

SPF is a commonly used measure of photoprotection of a sunscreen against erythema. This number is derived from another parameter, the minimal erythemal dose (MED). MED is defined as the "least exposure dose at a specified wavelength that will elicit a delayed erythema response". The MED indicates the amount of energy reaching the skin and the responsiveness of the skin to the radiation. The SPF of a particular photoprotector is obtained by dividing the MED of protected skin by the MED of unprotected skin. The higher the SPF, the more effective the agent in preventing sunburn. The SPF value tells how many times longer a person can stay in the sun with use of the sunscreen (compared to a person with unprotected skin) before that person will experience 1 MED. For example, utilizing a sunscreen with an SPF of 6 will allow an individual to stay in the sun six times longer before receiving 1 MED. As the SPF value of a sunscreen increases, the less chance exists for development of tanning of the skin. Commercially available sunscreening products have SPF values ranging from 2 to 50.

Also particularly useful along with ethylhexyl-p-methoxycinnamate are sunscreens such as those disclosed in Sabatelli, US-A-4,937,370 and Sabatelli et al., US-A-4,999,186. The sunscreening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range.

These sunscreening agents provide higher efficacy, broader UV absorption, lower skin penetration and longer lasting efficacy relative to conventional sunscreens.

Preferred members of this class of sunscreening agents are 4-N,N-(2-ethylhexyl)methylaminobenzoic acid ester of 2,4-dihydroxybenzophenone; N,N-di-(2-ethylhexyl)-4-aminobenzoic acid ester with 4-hydroxydibenzoylmethane; 4-N,N(2-ethylhexyl) methylaminobenzoic acid ester with 4-hydroxydibenzoylmethane; 4-N,N-(2-ethylhexyl)methylaminobenzoic acid ester of 2-hydroxy-4-(2-hydroxyethoxy)benzophenone; 4-N,N-(2-ethylhexyl) methylaminobenzoic acid ester of 4-(2-hydroxyethoxy)dibenzoylmethane; N-N-di-(2-ethylhexyl)-4-aminobenzoic acid ester of 2-hydroxy-4-(2-hydroxyethoxy)benzophenone, and N,N-di-(2-ethylhexyl)4-aminobenzoic acid ester of 4-(2-hydroxyethoxy)dibenzoylmethane and mixtures thereof.

Emulsifier. The compositions of the present invention also essentially comprise at least one emulsifier comprising polymers of a monomeric mixture selected from acrylic, methacrylic and ethacrylic acids, 1 to 3.5 percent of an acrylate ester of the formula

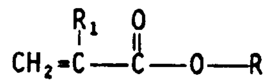

wherein R is hydrogen or an alkyl radical containing 10 to 30 carbon atoms and $R_1$ is hydrogen, methyl or ethyl, and 0.1 to 0.6 percent of a polymerizable cross-linking polyalkenyl polyether of a polyhydric alcohol containing more than one alkenyl ether group per molecule wherein the parent polyhydric alcohol contains at least 3 carbon atoms and at least 3 hydroxyl groups.

Preferably, these polymers contain from 96 to 97.9 percent of acrylic acid and from 2.5 to 3.5 percent of acrylic esters wherein the alkyl group contains 12 to 22 carbon atoms, and $R_1$ is methyl, most preferably the acrylate ester is stearyl methacrylate. Preferably, the amount of crosslinking monomer is from 0.2 to 0.4 percent. The preferred crosslinking monomers are allyl pentaerythritol, trimethylolpropane diallylether or allyl sucrose. These polymers are fully described in US-A-4,509,949, Huang et al., issued April 5, 1985.

The carboxylic monomers useful in the production of polymers used in this invention are the olefinically-unsaturated carboxylic acids containing at least one activated carbon-to-carbon olefinic double bond, and at least one carboxyl group.

The preferred carboxylic monomers are the acrylic acids having the general structure

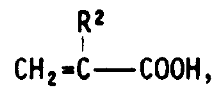

wherein $R^2$ is a substituent selected from the class consisting of hydrogen, halogen, and the cyanogen (--C=N) groups, monovalent alkyl radicals, monovalent alkaryl radicals and monovalent cycloaliphatic radicals. Of this class, acrylic, methacrylic, and ethacrylic acid are most preferred. Another useful carboxylic monomer is maleic anhydride or the acid. The amount of acid used will be from 95.5 to 98.9 percent of the total monomers used. More preferably the range will be from 96 to 97.9 percent.

The polymers are crosslinked with a polyfunctional vinylidene monomer containing at least 2 terminal $CH_2$< groups, including for example, butadiene, isoprene, divinyl benzene, divinyl naphthlene, allyl acrylates, and the like. Particularly useful crosslinking monomers for use in preparing the copolymers are polyalkenyl polyethers having more than one alkenyl ether grouping per molecule. The most useful possess alkenyl groups in which an olefinic double bond is present attached to a terminal methylene grouping, $CH_2$=C<. Most preferred is Carbomer 1342 (available as Carbopol (RTM) 1342 from B.F. Goodrich).

The emulsifier typically comprises in total from 0.01% to 2.0%, preferably from 0.1% to 0.75%, and most preferably from 0.2% to 0.5%, of the compositions of the present invention.

Wax Component. An essential component of the compositions herein is a non-polar wax component having a required HLB of from 1 to 8, more preferably from 1 to 7 and a melting point greater than 50°C, and preferably greater than 60°C.

Useful waxes include ester waxes, diester waxes, hydrocarbon waxes, silicone waxes and triglyceride waxes and mixtures thereof.

The HLB (short for "Hydrophile-Lipophile Balance") value system is fully described, and values for various materials are provided, in the publication The HLB System, A Time-Saving Guide to Emulsifier Selection (published by ICI Americas Inc., Wilmington, Delaware; 1984).

Useful ester waxes include $C_{16}$-$C_{40}$ alcohols esterfied with $C_{16}$-$C_{40}$ fatty acid, diesters of $C_{16}$-$C_{40}$ fatty acid where the alcohol is propylene glycol, ethylene glycol, polyethylene glycol, polypropylene glycol, $C_{12}$-$C_{40}$, triglycerides or $C_{18}$-$C_{40}$ diglycerides, pentaerythitol tri- or tetra- esters of $C_{12}$-$C_{40}$ fatty acids, $C_{18}$-$C_{40}$ fatty acids of sorbitan triesters. Useful synthetic waxes include Fischer-Tropsche waxes. Useful hydrocarbon waxes include paraffin, micro-crystalline waxes, and mixtures thereof. Useful diester waxes include Syncrowax (RTM) ERL-C (available from Croda); $C_{18}$-$C_{40}$ fatty acid diester and propylene glycol diester waxes including propylene glycol distearate and glycol distearate. Useful triglyceride waxes include Syncrowax (RTM) HGL-C, Syncrowax (RTM) HRC, Syncrowax (RTM) HRS-C (all available from Croda Inc.), tristearin, trimyristate, castor wax and fully hydrogenated vegetable or animal oils as well as alkyloxysilicone waxes (such as Dow Corning 580 Wax) and mixtures thereof.

Waxes useful in the compositions of this invention are disclosed in the following: US-A-4,049,792, to Elsnau, issued September 20, 1977; US-A-4,151,272, to Geary et al., issued April 24, 1975; US-A-4,229,432, to Geria, issued October 21, 1980; US-A-4,280,994, to Turney, issued July 28, 1981; US-A-4,126,679, to Davy et al., issued November 21, 1978; and EP-A-0,117,070, to May, published August 29, 1984, "The Chemistry and Technology of Waxes", A.H. Warth, 2nd Edition, reprinted in 1960, Reinhold Publishing Corporation, pp

391-393 and 421; "The Petroleum Chemicals Industry", R.F. Goldstein and A.L. Waddeam, 3rd Edition (1967), E & F.N. Span Ltd., pp 33-40; "The Chemistry and Manufacture of Cosmetics", M.G. DeNavarre, 2nd edition (1970), Van Nostrand & Company, pp 354-376; and in "Encyclopedia of Chemical Technology:, Vol. 24, Kirk-Othmer, 3rd Edition (1979) pp 466-481.

Film-forming Copolymer

The compositions of the present invention also essentially comprise a film-forming copolymer of the formula:

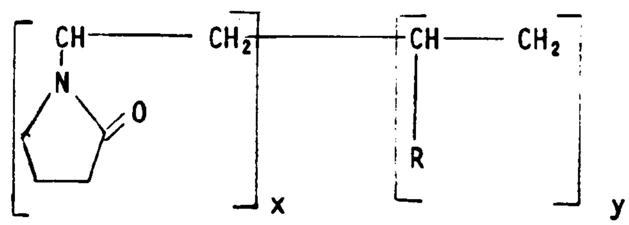

where R is a $C_{12}$-$C_{30}$ straight chain alkyl and preferably a $C_{18}$-$C_{22}$ straight chain alkyl and wherein the ratio of x to y is from 1:5 to 5:1. A particularly preferred copolymer is 1-eicosene polymer with 1-ethenyl-2-pyrrolidinone available from GAF Chemical Corporation as Ganex V-220®.

These film-forming copolymers are present at a level of from 0.1% to 5.0%, preferably from 0.1% to 2%.

Optional Components

Emollients. The compositions of the present invention preferably comprise at least one emollient. Useful emollients have a required HLB below 10. Preferred emollients are volatile silicone oils, non-volatile emollients, and the highly branched hydrocarbons known as the Permethyl (RTM) 99 through 108A series (available from Permethyl Corporation) and mixtures thereof. The compositions of the present invention more preferably comprise at least one volatile silicone oil which functions as a liquid emollient, or especially in a mixture of volatile silicone oils and non-volatile emollients. The term "volatile", as used herein, refers to those materials which have a measurable vapor pressure at ambient temperature.

Volatile silicone oils useful in the compositions of the present invention are preferably cyclic. The following formula illustrates cyclic volatile polydimethylsiloxanes useful in the compositions disclosed herein:

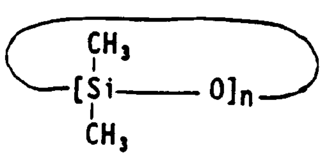

wherein n equals from 3 to 7. Linear polydimethylsiloxanes contain from 3 to 9 silicon atoms per molecule and have the following general formula:

$$(CH_3)_3Si\text{-}O\text{-}[Si(CH_3)_2\text{-}O]_n\text{-}Si(CH_3)_3$$

wherein n equals 1 to 7. Linear volatile silicone materials generally have viscosities of less than 5 $mm^2.s^{-1}$ (5 centistokes) at 25°C while cyclic materials typically have viscosities of less than 10 $mm^2.s^{-1}$ (10 centistokes). A description of various volatile silicone oils is found in Todd, et al., "Volatile Silicone Fluids for Cosmetics", Cosmetics & Toiletries, 91, pages 27-32 (1976).

Examples of preferred volatile silicone oils useful herein include: Dow Corning 344, Dow Corning 345, and Dow Corning 200 (manufactured by Dow Corning Corp.); Silicone 7207 and Silicone 7158 (manufactured by the Union Carbide Corp.); SF 1202 (manufactured by General Electric); and SWS-03314 (manufactured by SWS Silicones, Inc.).

The present compositions also preferably contain one or more non-volatile emollients. Such materials include fatty acid and fatty alcohol esters, hydrocarbons, non-volatile silicone oils, and mixtures thereof. Emollients among those useful herein are described in Cosmetics, Science and Technology 27-104 (M. Balsam and E. Sagarin, Ed.; 1972), and US-A-4,202,879, to Shelton, issued May 13, 1980.

Non-volatile silicone oils useful as an emollient material include polyalkylsiloxanes and polyalklyarylsiloxanes. The essentially non-volatile polyalkyl siloxanes useful herein include, for example, polydimethyl siloxanes with viscosities of from 5 to 100 000 $mm^2.s^{-1}$ (100,000 centistokes) at 25°C. Among the preferred non-volatile emollients useful in the present compositions are the polydimethyl siloxanes having viscosities from 10 $mm^2.s^{-1}$ (10 centistokes) to 400 $mm^2.s^{-1}$ (400 centistokes) at 25°C. Such polyalkyl siloxanes include the Vicasil (RTM) series (sold by General Electric Company) and the Dow Corning 200 series (sold by Dow Corning Corporation). Polyalkylaryl siloxanes include poly methylphenyl siloxanes having viscosities of from 15 $mm^2.s^{-1}$ (15 centistokes) to 65 $mm^2.s^{-1}$ (65 centistokes) at 25°C. These are available, for example, as SF 1075 methylphenyl fluid (sold by General Electric Company) and 556 Cosmetic Grade Fluid (sold by Dow Corning Corporation).

Non-polar fatty acid and fatty alcohol esters useful herein as an emollient material include, for example, ethyl hexyl palmitate, isodecyl neopentanoate, octadodecyl benzoate, diethyl hexyl maleate and PPG-2 myristyl ether propionate. Hydrocarbons such as isohexadecane (e.g., Permethyl (RTM) 101A supplied by Preperse), petrolatum and USP light (e.g. Klearol®) or heavy (e.g. Kaydol®) mineral oils are also useful as emollients.

The emollients typically comprise in total from 1% to 50%, preferably from 1% to 25%, and more preferably from 1% to 10% by weight of the compositions of the present invention.

The compositions of the present invention may also contain in addition to the aforementioned components, a wide variety of additional oil soluble materials and/or water soluble materials.

Other oil soluble materials include esters such as cetearyl palmitate, lauryl myristate and isopropyl palmitate; oils such as castor oil, jojoba oil, cottonseed oil, peanut oil and sesame oil; waxes such as petrolatum, ceresin wax, carnauba wax, beeswax, and castor wax; cetyl palmitate and glyceryl tribehenate; oil dispersable powders such as aluminum starch octenyl succinate. Sterols such as soya sterol, cholesterol and phytosterol are also useful herein. Highly preferred for use herein are isodecyl neopentanoate, isoarachidyl neopentanoate, and isohexadecane and mixtures thereof.

These optional oil phase materials may individually comprise up to 20% by weight of the total sunscreen composition, preferably from 10% to 15%.

Additional water soluble materials may also be present in the compositions of this invention. Included are humectants such as glycerine, hexylene glycol, sorbitol, propylene glycol, alkoxylated glucose and hexanetriol; thickening agents; hydroxyethyl cellulose, carboxymethyl cellulose, vegetable gums and clays such as Veegum® (magnesium aluminum silicate, R. T. Vanderbilt, Inc.); proteins and polypeptides; preservatives such as the methyl, ethyl, propyl and butyl esters of hydroxybenzoic acid (Parabens - Mallinckrodt Chemical Corp.), EDTA, methylisothiazolinone and imidazolidinyl ureas (Germall (RTM) 115 - Sutton Laboratories); and pH controlling agents such as triethanolamine, sodium hydroxide, potassium hydroxide or citric acid, if desired. Additional materials include UV scattering powders (e.g., titanium dioxide) and antioxidants such as propylene glycol (and) BHA (and) propyl gallate (and) citric acid, as well as chelators such as disodium EDTA.

The water phase materials may individually comprise up to 25% by weight of the total sunscreen composition, preferably up to 15%.

The present compositions may also contain agents suitable for aesthetic purposes such as perfumes and/or dyes.

The pH of the sunscreen compositions herein is preferably in the range of from 4.5 to 9.

For an aqueous emulsion sunscreen composition of the present invention, the mean particle size of the dispersed oil phase materials (e.g., sunscreen agent, polymer, perfumes, etc.) dispersed in the aqueous phase may be in the range of from 5 μm to 10 μm with greater than 75% of the particles being less than 12 μm.

Also useful in levels that total less than 0.5% are surfactants such as TEA stearate salts, alkali neutralized mono- and di-alkyl phosphates including diethanolamine mono- and di- cetyl phosphate (available from Givaudan as Amphisol (RTM)) and hydroxy cetyl phosphate (available from Henkel as Forlanit (RTM) E). Of course, the emulsion must maintain a surface tension greater than 0.04 N/m (40 dynes/cm).

The pharmaceutically-acceptable sunscreen carriers, in total, typically comprise from 0.1% to 99.8% by weight of the sunscreen compositions of the present invention, preferably from 80% to 99%, and most preferably from 85% to 95%.

The compositions of the present invention may be prepared using the method described in the examples hereinafter.

## EXAMPLE I

An oil-in-water emulsion prepared by combining the following components utilizing conventional mixing techniques.

**Water Phase**

| Ingredients | % Weight |
|---|---|
| Water | 82.05 |
| Carbomer 1342[1] | 0.20 |
| Carbomer 954[2] | 0.30 |
| Butylene Glycol | 2.00 |
| Methyl paraben | 0.25 |
| Disodium EDTA | 0.075 |

**Oil Phase**

| Ingredients | % Weight |
|---|---|
| Ethylhexyl-p-methoxycinnamate[3] | 7.50 |
| Isoarchidyl neopentanoate[4] | 2.00 |
| Polyvinylpyrollidone/eicosene copolymer | 1.50 |
| Glyceryl tribehenate[5] | 1.50 |
| Stearoxy trimethylsilane/stearyl alcohol[6] | 0.50 |
| Propylparaben | 0.15 |

[1] Available as Carbopol (RTM) 1342 from Union Carbide.
[2] Available as Carbopol (RTM) 954 from Union Carbide.
[3] Available as Parsol (RTM) MCX from Givaudan Corporation.
[4] Available as Elefac (RTM) I-205 from Bernel Chemical.
[5] Available as Syncrowax (RTM) HRC from Croda.
[6] Available as DC 580 Wax from Dow Corning.

In a suitable vessel, combine the water phase ingredients and heat to 80°C. The oil phase ingredients are then combined and added to the water phase under high shear at 80°C. 1.0% water and 0.625% triethanolamine are then added to this mixture. The resulting mixture is cooled to 40°C and 0.3% of a preservative (imidazolidinyl urea, avaliable as Germall (RTM) 115 from Sutton Laboratories) is added.

This emulsion is useful for topical application to inhibit damage caused by acute or chronic UV exposure. Use of an amount of this composition sufficient to deposit about 2.0 mg/cm$^2$ of the sunscreening agent to the skin prior to UV exposure is appropriate.

EXAMPLES II-IV

| Ingredients | % w/w | | |
|---|---|---|---|
| | Ex. II | Ex. III | Ex. IV |
| Water, purified | q.s. | q.s. | q.s. |
| Carbomer 1342 | 0.09 | 0.10 | 0.14 |
| Carbomer 954 | -- | -- | 0.21 |
| Carbomer 934 | 0.135 | -- | -- |
| Carbomer 940 | -- | 0.15 | -- |
| Triethanolamine | 0.28 | 0.31 | 0.50 |
| Ethylhexyl-p-methoxycinnamate | 5.50 | 1.00 | 7.50 |
| Padimate O[1] | 6.00 | 6.00 | -- |
| Benzophenone-3 | -- | 1.00 | -- |
| Isodecyl neopentanoate | -- | 3.00 | -- |
| Isoarchidyl neopentanoate | 10.00 | -- | -- |
| Glyceryl tri-octanoate[2] | -- | -- | 10.00 |
| Polyvinylpropyrollidone/eicosene copolymer | 1.00 | 0.5 | 0.5 |
| Glyceryl tribehenate | -- | 1.00 | -- |
| Cetyl palmitate | -- | 1.00 | 2.00 |
| Tetrabehenate | 2.00 | -- | -- |
| Butylene glycol | 1.00 | 0.50 | -- |
| Hexylene glycol | -- | -- | 1.00 |
| Imidazolidinyl urea | -- | 0.30 | 0.30 |
| DMDM Hydantoin[3] | 0.30 | -- | -- |
| Methyl paraben | 0.250 | 0.250 | 0.250 |
| Propyl paraben | 0.150 | 0.150 | 0.150 |
| Disodium EDTA | 0.10 | 0.075 | 0.10 |
| Butylated hydroxy anisol[4] | -- | 0.050 | -- |
| DEA cetyl phosphate[5] | -- | 0.20 | -- |
| Fragrance | 0.20 | -- | 0.10 |

[1] Available as Escalol (RTM) 507 from Van Dyke.

[2] Available as Trivent (RTM) OCG from Trivent Chemical Co., Inc.

[3] Available as Glydant (RTM) from Glycol.

[4] Available as BHA tablets from Eastman Chemical Products lnc.

[5] Available as Amphisol (RTM) from Bernel Chemical.

## Claims

1. An oil-in-water sunscreen emulsion composition having improved efficiency for topical use comprising:
(a) from 1% to 20% of ethylhexyl-p-methoxycinnamate;
(b) from 0.025% to 2.0% of a carboxylic copolymer comprising polymers of a monomeric mixture selected from acrylic, methacrylic and ethacrylic acids, 1 to 3.5 weight percent of an acrylate ester of the formula:

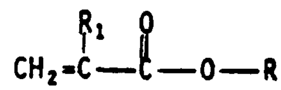

wherein R is hydrogen or an alkyl radical containing 10 to 30 carbon atoms and $R_1$ is hydrogen, methyl or ethyl, and 0.1 to 0.6 weight percent of a polymerizable cross-linking polyalkenyl polyether of a polyhydric alcohol containing more than one alkenyl ether group per molecule wherein the parent polyhydric alcohol contains at least 3 carbon atoms and at least 3 hydroxyl groups;
(c) from 0.1% to 5.0% of a film-forming copolymer of the formula:

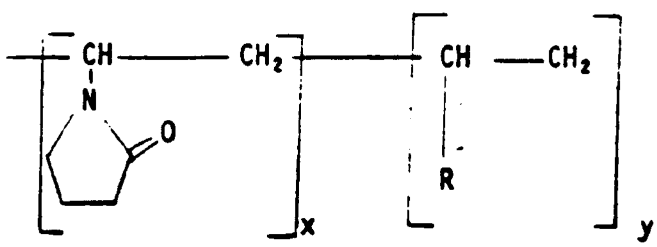

where R is a $C_{12}$-$C_{30}$ straight chain alkyl and wherein the ratio of x to y is from 1:5 to 5:1;
(d) from 0.1% to 10% of a wax component having a required HLB of from 1 to 8 and a melting point greater than 50°C; and
(e) a safe and effective amount of a topical carrier;
wherein said sunscreen emulsion has a surface tension greater than 0.04 N/m (40 dynes/cm) and wherein the oil phase has a required HLB between 1 and 8.

2. A sunscreen composition according to Claim 1 wherein said wax component is selected from ester waxes, hydrocarbon waxes, diester waxes, silicone waxes and triglyceride waxes and mixtures thereof.

3. A sunscreen composition according to Claim 2 wherein said wax component has a melting point greater than 60°C and is present at a level of from 5% to 35%.

4. A sunscreen composition according to Claim 3 wherein said ester wax is selected from $C_{16}$-$C_{40}$ alcohols esterfied with $C_{16}$-$C_{40}$ fatty acid, diesters of $C_{16}$-$C_{40}$ fatty acid where the alcohol is propylene glycol, ethylene glycol, polyethylene glycol, polypropylene glycol, $C_{12}$-$C_{40}$, triglycerides or $C_{18}$-$C_{40}$ diglycerides, pentaerythitol tri- or tetra- esters of $C_{12}$-$C_{40}$ fatty acids, $C_{18}$-$C_{40}$ fatty acids of sorbitan triesters and mixtures thereof.

5. A sunscreen composition according to Claim 4 wherein said wax component has a required HLB of from 1 to 7.

6. A sunscreen composition according to Claim 5 wherein said diester wax is selected from $C_{18-36}$ fatty acid glycol ester; $C_{18}$-$C_{40}$ fatty acid diester and propylene glycol diester waxes including propylene glycol distearate and glycol distearate and mixtures thereof.

7. A sunscreen composition according to Claim 6 wherein said triglyceride wax is selected from $C_{18-36}$ fatty acid triglyceride, 1,2,3-Propanetriol ester of behenic acid, tristearin, trimyristate, castor wax, fully hydrogenated vegetable oils alkyloxysilicone waxes and mixtures thereof.

8. A sunscreen composition according to Claim 7 wherein said film-forming copolymer is 1-eicosene polymer

with 1-ethenyl-2-pyrrolidinone.

**9.** A sunscreen composition according to Claim 8 which further comprises from 1% to 30% of an additional sunscreen active and wherein said sunscreen active is selected from 2-ethylhexyl p-methoxycinnamate, butylmethoxydibenzoylmethane, 2-hydroxy-4-methoxybenzophenone, 2,2' dihydroxy-4-methoxybenzophenone and ethyl hexyl salicylate, octyldimethyl p-aminobenzoic acid, the 4-N,N-(2-ethylhexyl)-methylaminobenzoic acid ester of 2,4-dihydroxybenzophenone, the N,N-di-(ethylhexyl)-4-aminobenzoic acid ester of 4-hydroxydibenzoylmethane, the 4-N,N-(2-ethylhexyl)methylaminobenzoic acid ester of 4-hydroxydibenzoylmethane, the 4-N,N-(2-ethylhexyl)-methylaminobenzoic acid ester of 2-hydroxy-4-(2-hydroxyethoxy) benzophenone, the 4-N,N-(2-ethylhexyl)methylaminobenzoic acid ester of 4-(2-hydroxyethoxy) dibenzoylmethane, the N-N-di-(2-ethylhexyl)-4-aminobenzoic acid ester of 2-hydroxy-4-(2-hydroxyethoxy)benzophenone, or the N.N-di-(2-ethylhexyl)-4-aminobenzoic acid ester of 4-(2-hydroxyethoxy)dibenzoylmethane and mixtures thereof.

**10.** A sunscreen composition according to Claim 9 wherein said sunscreen active is selected from 2-ethylhexyl p-methoxycinnamate, butylmethoxydibenzoylmethane, 2-hydroxy 4-methoxybenzophenone, octyldimethyl p-aminobenzoic acid and mixtures thereof.

**11.** A sunscreen composition according to Claim 8 which further comprises from 1% to 30% of an additional sunscreen active and wherein said sunscreen active is selected from 4-N,N-(2-ethylhexyl)methylaminobenzoic acid ester of 2,4-dihydroxybenzophenone; N,N-di-(2-ethylhexyl)-4-aminobenzoic acid ester with 4-hydroxydibenzoylmethane; 4-N,N(2-ethylhexyl) methylaminobenzoic acid ester with 4-hydroxydibenzoylmethane; 4-N,N-(2-ethylhexyl)methylaminobenzoic acid ester of 2-hydroxy-4-(2-hydroxyethoxy)benzophenone; 4-N,N-(2-ethylhexyl) methylaminobenzoic acid ester of 4-(2-hydroxyethoxy)dibenzoylmethane; N-N-di-(2-ethylhexyl)-4-aminobenzoic acid ester of 2-hydroxy-4-(2-hydroxyethoxy)benzophenone, and N,N-di-(2-ethylhexyl)4-aminobenzoic acid ester of 4-(2-hydroxyethoxy)-dibenzoylmethane and mixtures thereof.

## Patentansprüche

**1.** Sonnenschutzzusammensetzung auf der Basis einer Öl-in-Wasser-Emulsion, welche Zusammensetzung eine verbesserte Wirksamkeit für die topische Anwendung aufweist, und welche enthält:

(a) 1 % bis 20 % Ethylhexyl-p-methoxycinnamat;

(b) 0,025 % bis 2,0 % eines Carboxylsäurecopolymers, welches Polymere aus einem Gemisch von Monomeren, welche aus Acryl-, Methacryl- und Ethacrylsäure ausgewählt sind, 1 bis 3,5 Gew.-% eines Acrylatesters der Formel:

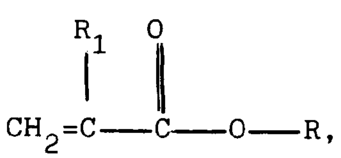

worin R für Wasserstoff oder einen Alkylrest mit 10 bis 30 Kohlenstoffatomen steht und $R_1$ für Wasserstoff, Methyl oder Ethyl steht, sowie 0,1 bis 0,6 Gew.-% eines polymerisierbaren, vernetzenden Polyalkenylpolyethers eines mehrwertigen Alkohols, welcher mehr als eine Alkenylethergruppe pro Molekül enthält, und wobei der mehrwertige Stammalkohol wenigstens 3 Kohlenstoffatome und wenigstens 3 Hydroxylgruppen enthält:

(c) 0,1 % bis 5,0 % eines filmbildenden Copolymers der Formel:

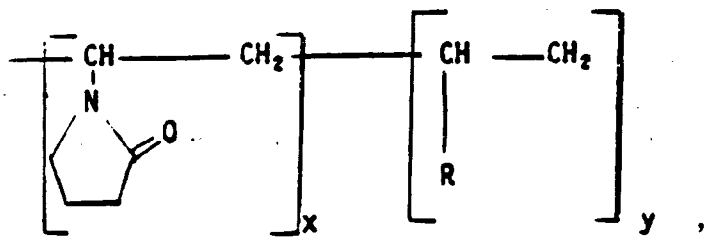

worin R eine geradkettige $C_{12}$-$C_{30}$-Alkylgruppe ist und worin das Verhältnis von x zu y 1:5 bis 5:1 beträgt;
(d) 0,1 bis 10 % einer Wachskomponente, welche einen erforderlichen HLB-Wert von 1 bis 8 und einen Schmelzpunkt von über 50°C aufweist, und
(e) eine sichere und wirksame Menge eines topischen Trägers;
wobei diese Sonnenschutzemulsion eine Oberflächenspannung von über 0,04 N/m (40 dyn/cm) aufweist und wobei die Ölphase einen erforderlichen HLB-Wert zwischen 1 und 8 aufweist.

2. Sonnenschutzzusammensetzung nach Anspruch 1, wobei die Wachskomponente aus Esterwachsen, Kohlenwasserstoffwachsen, Diesterwachsen, Silikonwachsen und Triglyceridwachsen und Gemischen hievon ausgewählt ist.

3. Sonnenschutzzusammensetzung nach Anspruch 2, wobei die Wachskomponente einen Schmelzpunkt von über 60°C aufweist und in einer Menge von 5 % bis 35 % vorliegt.

4. Sonnenschutzzusammensetzung nach Anspruch 3, wobei das Esterwachs aus mit einer $C_{16}$-$C_{40}$-Fettsäure veresterten $C_{16}$-$C_{40}$-Alkoholen, Diestern einer $C_{16}$-$C_{40}$-Fettsäure, wobei der Alkohol Propylenglykol, Ethylenglykol, Polyethylenglykol, Polypropylenglykol ist, $C_{12}$-$C_{40}$-Triglyceriden oder $C_{18}$-$C_{40}$-Diglyceriden, Pentaerythrittri- oder -tetraestern von $C_{12}$-$C_{40}$-Fettsäuren, $C_{18}$-$C_{40}$-Fettsäuren von Sorbitantriestern und Gemischen hievon ausgewählt ist.

5. Sonnenschutzzusammensetzung nach Anspruch 4, wobei die Wachskomponente einen erforderlichen HLB-Wert von 1 bis 7 aufweist.

6. Sonnenschutzzusammensetzung nach Anspruch 5, wobei das Diesterwachs aus $C_{18-36}$-Fettsäureglykolester; $C_{18}$-$C_{40}$-Fettsäurediester- und Propylenglykoldiesterwachsen,einschließlich des Propylenglykoldistearates und Glykoldistearates und Gemischen hievon ausgewählt ist.

7. Sonnenschutzzusammensetzung nach Anspruch 6,wobei das Triglyceridwachs aus $C_{18-36}$-Fettsäuretriglycerid, dem 1,2,3-Propantriolester von Behensäure, Tristearin, Trimyristat, Rizinuswachs,vollständig hydrierten pflanzlichen Ölen, Alkyloxysilikonwachsen und Gemischen hievon ausgewählt ist.

8. Sonnenschutzzusammensetzung nach Anspruch 7, wobei das filmbildende Copolymer das 1-Eicosenpolymer mit 1-Ethenyl-2-pyrrolidinon ist.

9. Sonnenschutzzusammensetzung nach Anspruch 8, welche weiterhin 1 % bis 30 % eines weiteren Sonnenschutzwirkstoffes enthält, wobei dieser Sonnenschutzwirkstoff aus 2-Ethylhexyl-p-methoxycinnamat,Butylmethoxydibenzoylmethan, 2-Hydroxy-4-methoxybenzophenon, 2,2'-Dihydroxy-4-methoxybenzophenon und Ethylhexylsalicylat, Octyldimethyl-p-aminobenzoesäure, dem 4-N,N-(2-Ethylhexyl)methylaminobenzoesäureester von 2,4-Dihydroxybenzophenon, dem N,N-Di-(ethylhexyl)-4-aminobenzoesäureester von 4-Hydroxydibenzoylmethan, dem 4-N,N-(2-Ethylhexyl)methylaminobenzoesäureester von 4-Hydroxydibenzoylmethan, dem 4-N,N-(2-Ethylhexyl)methylaminobenzoesäureester von 2-Hydroxy-4-(2-hydroxyethoxy)benzophenon, dem 4-N,N-(2-Ethylhexyl)methylaminobenzoesäureester von 4-(2-Hydroxyethoxy)-dibenzoylmethan, dem N,N-Di-(2-ethylhexyl)-4-aminobenzoesäureester von 2-Hydroxy-4-(2-hydroxyethoxy)benzophenon, oder dem N,N-Di-(2-ethylhexyl)-4-aminobenzoesäureester von 4-(2-Hydroxyethoxy)dibenzoylmethan und Gemischen hievon ausgewählt ist.

10. Sonnenschutzzusammensetzung nach Anspruch 9, wobei der Sonnenschutzwirkstoff aus 2-Ethylhexyl-p-methoxycinnamat, Butylmethoxydibenzoylmethan, 2-Hydroxy-4-methoxybenzophenon, Octyldimethyl-p-aminobenzoesäure und Gemischen hievon ausgewählt ist.

11. Sonnenschutzzusammensetzung nach Anspruch 8, welche weiterhin 1 % bis 30 % eines weiteren Sonnenschutzwirkstoffes enthält, wobei dieser Sonnenschutzwirkstoff aus dem 4-N,N-(2-Ethylhexyl)methylaminobenzoesäureester von 2,4-Dihydroxybenzophenon; dem N,N-Di-(2-ethylhexyl)-4-aminobenzoesäureester mit 4-Hydroxydibenzoylmethan; dem 4-N,N-(2-Ethylhexyl)-methylaminobenzoesäureester mit 4-Hydroxydibenzoylmethan;dem 4-N,N-(2-Ethylhexyl)methylaminobenzoesäureester von 2-Hydroxy-4-(2-hydroxyethoxy)benzophenon; dem 4-N,N-(2-Ethylhexyl)-methylaminobenzoesäureester von 4-(2-Hydroxyethoxy)dibenzoylmethan; dem N-N-Di-(2-ethylhexyl)-4-aminobenzoesäureester von 2-Hydroxy-4-(2-hydroxyethoxy)benzophenon, und dem N,N-Di-(2-ethylhexyl)4-aminobenzoesäureester von 4-(2-Hydroxyethoxy)dibenzoylmethan und Gemischen hievon ausgewählt ist.

## Revendications

1. Composition d'écran anti-solaire en émulsion huile dans l'eau à usage local, comprenant :
   (a) de 1% à 20% de p-méthoxycinnamate d'éthylhexyle;
   (b) de 0,025% à 2,0% d'un copolymère carboxylique comprenant des polymères d'un mélange monomère choisi parmi les acides acryliques, méthacryliques et éthacryliques, de 1 à 3,5% en poids d'un acrylate de formule :

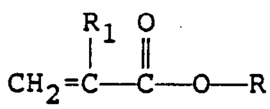

   dans laquelle R est un hydrogène ou un radical alkyle contenant 10 à 30 atomes de carbone et $R_1$ est un hydrogène ou un radical méthyle ou éthyle, et de 0,1 à 0,6% en poids d'un polyéther polyalcénylique de réticulation polymérisable d'un polyol contenant plus d'un groupe alcényloxy par molécule, dans lequel la molécule mère de polyol contient au moins 3 atomes de carbone et au moins 3 groupes hydroxyle;
   (c) de 0,1% à 5,0% d'un copolymère filmogène de formule :

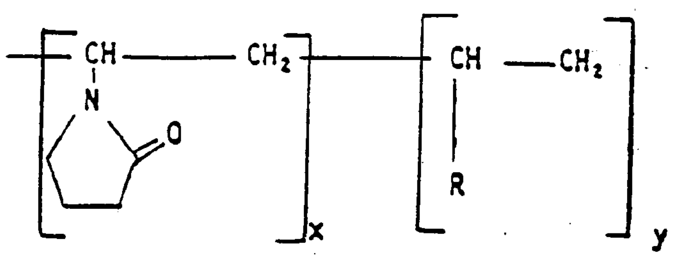

   dans laquelle R est un radical alkyle à chaîne droite en $C_{12}$-$C_{30}$ et dans laquelle le rapport de x à y est de 1 : 5 à 5 : 1.
   (d) de 0,1% à 10% d'un constituant cireux possédant un rapport hydrolipophile (HLB) spécifié de 1 à 8 et un point de fusion supérieur à 50°C; et
   (e) une quantité sans danger et efficace d'un véhicule à usage local;
   dans laquelle ladite émulsion d'écran anti-solaire a une tension superficielle supérieure à 0,04 N/m (40 dynes/cm) et dans laquelle la phase huileuse a un HLB spécifié compris entre 1 et 8.

2. Composition d'écran anti-solaire selon la revendication 1, dans laquelle ledit constituant cireux est choisi parmi les cires d'esters, les cires d'hydrocarbures, les cires de diesters, les cires de silicones et les cires de triglycérides, et les mélanges de celles-ci.

3. Composition d'écran anti-solaire selon la revendication 2, dans laquelle ledit constituant cireux a un point de fusion supérieur à 60°C et est présent en proportion de 5% à 35%.

4. Composition d'écran anti-solaire selon la revendication 3, dans laquelle ladite cire d'ester est choisie parmi les alcools en $C_{16}$-$C_{40}$ estérifiés avec un acide gras en $C_{16}$-$C_{40}$, les diesters d'acides gras en $C_{16}$-$C_{40}$ dans

lesquels l'alcool est le propylèneglycol, l'éthylèneglycol, le polyéthylèneglycol, le polypropylèneglycol, les triglycérides en $C_{12}$-$C_{40}$ ou les diglycérides en $C_{18}$-$C_{40}$, les tri-esters ou tétra-esters de pentaérythritol et d'acides gras en $C_{12}$-$C_{40}$, les tri-esters de sorbitanne et d'acides gras en $C_{18}$-$C_{40}$, et les mélanges de ceux-ci.

5. Composition d'écran anti-solaire selon la revendication 4, dans laquelle ledit constituant cireux possède un HLB spécifié de 1 à 7.

6. Composition d'écran anti-solaire selon la revendication 5, dans laquelle ladite cire de diester est choisie parmi les esters de glycol et d'acides gras en $C_{18}$-$C_{36}$, les cires de diesters d'acides gras en $C_{18}$-$C_{40}$ et les cires de diester de propylèneglycol, notamment le distéarate de propylèneglycol et le distéarate de glycol, et les mélanges de ceux-ci.

7. Composition d'écran anti-solaire selon la revendication 6, dans laquelle ladite cire de triglycéride est choisie parmi les triglycérides d'acides gras en $C_{18}$-$C_{36}$, l'ester de 1,2,3-propanetriol et d'acide béhénique, la tristéarine, le trimyristate, la cire de ricin, les huiles végétales totalement hydrogénées, les cires d'alkyloxysilicone et les mélanges de ceux-ci.

8. Composition d'écran anti-solaire selon la revendication 7, dans laquelle ledit copolymère filmogène est un polymère de 1-éicosène avec la 1-éthényl-2-pyrrolidinone.

9. Composition d'écran anti-solaire selon la revendication 8, qui comprend aussi de 1% à 30% d'une substance active anti-solaire supplémentaire et dans laquelle ladite substance active anti-solaire est choisie parmi le p-méthoxycinnamate de 2-éthylhexyle, le butylméthoxydibenzoylméthane, la 2-hydroxy-4-méthoxybenzophénone, la 2,2'-dihydroxy-4-méthoxybenzophénone et le salicylate d'éthylhexyle, l'acide octyldiméthyl-p-aminobenzoïque, l'ester d'acide 4-N,N-(2-éthylhexyl)méthylaminobenzoïque et de 2,4-dihydroxybenzophénone, l'ester d'acide N,N-di(éthylhexyl)-4-aminobenzoïque et de 4-hydroxydibenzoylméthane, l'ester d'acide 4-N,N-(2-éthylhexyl)méthylaminobenzoïque et de 4-hydroxydibenzoylméthane, l'ester d'acide 4-N,N-(2-éthylhexyl)méthylaminobenzoïque et de 2-hydroxy-4-(2-hydroxyéthoxy)benzophénone, l'ester d'acide 4-N,N-(2-éthylhexyl)méthylaminobenzoïque et de 4-(2-hydroxyéthoxy)dibenzoylméthane, l'ester d'acide N,N-di(2-éthylhexyl)-4-aminobenzoïque et de 2-hydroxy-4-(2-hydroxyéthoxy)benzophénone ou l'ester d'acide N,N-di(2-éthylhexyl)-4-aminobenzoïque et de 4-(2-hydroxyéthoxy)-dibenzoylméthane, et les mélanges de ceux-ci.

10. Composition d'écran anti-solaire selon la revendication 9, dans laquelle ladite substance active anti-solaire est choisie parmi le p-méthoxycinnamate de 2-éthylhexyle, le butylméthoxydibenzoylméthane, la 2-hydroxy-4-méthoxybenzophénone, l'acide octyldiméthyl-p-aminobenzoïque et les mélanges de ceux-ci.

11. Composition d'écran anti-solaire selon la revendication 8, qui comprend aussi de 1% à 30% d'une substance active anti-solaire supplémentaire et dans laquelle ladite substance active anti-solaire est choisie parmi l'ester d'acide 4-N,N-(2-éthylhexyl)méthylaminobenzoïque et de 2,4-dihydroxybenzophénone, l'ester d'acide N,N-di(2-éthylhexyl)-4-aminobenzoïque et de 4-hydroxydibenzoylméthane, l'ester d'acide 4-N,N-(2-éthylhexyl)méthylaminobenzoïque et de 4-hydroxydibenzoylméthane, l'ester d'acide 4-N,N-(2-éthylhexyl)méthylaminobenzoïque et de 2-hydroxy-4-(2-hydroxyéthoxy)benzophénone, l'ester d'acide 4-N,N-(2-éthylhexyl)méthylaminobenzoïque et de 4-(2-hydroxyéthoxy)dibenzoylméthane, l'ester d'acide N,N-di(2-éthylhexyl)-4-aminobenzoïque et de 2-hydroxy-4-(2-hydroxyéthoxy)benzophénone et l'ester d'acide N,N-di(2-éthylhexyl)-4-aminobenzoïque et de 4-(2-hydroxyéthoxy)dibenzoylméthane, et les mélanges de ceux-ci.